Europäisches Patentamt

European Patent Office

Office européen des brevets

(19)

(11) Veröffentlichungsnummer: **0 089 682**
**A1**

(12) # EUROPÄISCHE PATENTANMELDUNG

(21) Anmeldenummer: 83102852.7

(22) Anmeldetag: 22.03.83

(51) Int. Cl.³: **A 61 B 10/00**
**G 10 K 11/00**

(30) Priorität: 23.03.82 DE 3210610
24.01.83 DE 3302254

(43) Veröffentlichungstag der Anmeldung:
28.09.83 Patentblatt 83/39

(84) Benannte Vertragsstaaten:
AT CH DE FR GB LI

(71) Anmelder: SIEMENS AKTIENGESELLSCHAFT
Berlin und München Wittelsbacherplatz 2
D-8000 München 2(DE)

(72) Erfinder: Pirschel, Jörg, Dr.
Zwehrenbühlstrasse 44
D-7400 Tübingen(DE)

(54) **Zusatzeinrichtung zu einem handelsüblichen Ultraschall-Untersuchungsgerät, insbesondere zur Untersuchung der weiblichen Brust oder des Hodens.**

(57) Das zu untersuchende Körperteil (4) ragt über eine Öffnung (3) in einen Flüssigkeitsbehälter (6), der eine Ultraschallwellen gut leitende Flüssigkeit enthält. Das Körperteil ist an ein handelsübliches Echtzeit-Ultraschall-Abtastsystem (12) angekoppelt, das auch für andere Zwecke eingesetzt werden kann. Um Probleme hinsichtlich der Abdichtung und der elektrischen Isolierung zu vermeiden, ist das Abtastsystem (12) außerhalb des Behälters (6) angeordnet. Eine Haltevorrichtung (16, 18 - 20) hält das Abtastsystem (12) an der Ankoppelstelle außerhalb des Behälters (6) lösbar fest. Eine motorisch betriebene Einrichtung (33) dreht die Haltevorrichtung und das Abtastsystem gemeinsam um eine Rotationsachse (38), die auf das Körperteil weist und vorzugsweise vertikal steht. Bei der Rotation um das zu untersuchende Körperteil ist die Abstrahlfläche des Abtastsystems (12) bezüglich der Rotationsachse (38) schräg ausgerichtet. Der Flüssigkeitsbehälter kann z.B. durch einen Sack, durch einen Zylinder (64) oder durch eine Flüssigkeitsvorlaufstrecke (112, 112a) gebildet sein.

FIG 1

EP 0 089 682 A1

SIEMENS AKTIENGESELLSCHAFT          Unser Zeichen
Berlin und München                  VPA 82 P 8510 E kb

Zusatzeinrichtung zu einem handelsüblichen Ultraschall-Untersuchungsgerät, insbesondere zur Untersuchung der weiblichen Brust oder des Hodens

Die Erfindung bezieht sich auf eine Zusatzeinrichtung zur Untersuchung von Körperteilen, insbesondere der weiblichen Brust oder des Hodens, gemäß den Merkmalen des Oberbegriffes des Patentanspruches 1.

Eine Zusatzeinrichtung dieser Art ist aus der europäischen Patentanmeldung 00 32 751 bekannt. Eine Zusatzeinrichtung hat den Vorzug, daß der eigentliche Ultraschallkopf noch für andere Zwecke eingesetzt werden kann. Für die Mamma- und Hodenuntersuchung kommt man, sofern ein handelsüblicher Ultraschallkopf verwendet werden kann, ohne Spezialgerät aus, das ja nur für beschränkte spezielle Aufgaben einsetzbar ist. Solche Sonder- oder Spezialgeräte sind in der Regel teuer.

Eine Vorrichtung zur Ultraschalluntersuchung ist z.B. aus der WO-OS 80/00193 vorbekannt. Bei dieser Vorrichtung, die speziell zur Untersuchung der weiblichen Brust (Ultraschall-Mammadiagnostik) dient, ist ein den eigentlichen Flüssigkeitsbehälter umgebender zweiter Behälter Auffangbehälter für Flüssigkeit, die beim Eintauchen der weiblichen Brust aus dem Flüssigkeitsbehälter überläuft. Das Ultraschall-Abtastsystem sitzt direkt im Flüssigkeitsbehälter. Dies wirft jedoch spezielle Probleme hinsichtlich der Abdichtung und der elektrischen Isolierung auf.

Nm 2 Rl / 21.03.1983

Ähnliche Vorrichtungen, bei denen das Ultraschall-Abtastsystem ebenfalls in der Flüssigkeit des Flüssigkeitsbehälters untergebracht ist, bei denen allerdings der Flüssigkeitsbehälter nicht mit einem speziellen Überlaufbehälter umgeben ist, sind beispielsweise aus der DE-OS 29 19 995, der GB-OS 20 15 732 und aus der US-PS 41 05 018 vorbekannt. Auch bei diesen Vorrichtungen ergeben sich wegen des in der Flüssigkeit plazierten Ultraschall-Systems dieselben Probleme hinsichtlich Dichtigkeit und Isolierung.

Aufgabe vorliegender Erfindung ist es, eine Vorrichtung zu schaffen, die im Hinblick auf das Ultraschall-Abtastsystem keine Probleme hinsichtlich der Abdichtung und der elektrischen Isolierung aufwirft. Weiterhin soll ein übliches Ultraschall-Echtzeit-Echogerät für die Mamma- und/oder Hoden-Sonographie nachrüstbar sein. Es soll also ein entsprechendes Zusatzgerät geschaffen werden, das es gestattet, zusammen mit dem handelsüblichen Ultraschallgerät für Realtime-Bilddarstellung quantitative Diagnostik durchzuführen.

Die Aufgabe wird erfindungsgemäß durch die kennzeichnenden Merkmale des Anspruchs 1 gelöst.

Gemäß der Erfindung sitzt das Ultraschall-Abtastsystem außerhalb des eigentlichen Flüssigkeitsbehälters, z.B. in einem weiteren Behälter, der eine Schutzfunktion erfüllt. Das Ultraschall-Abtastsystem braucht also nicht extra abgedichtet zu werden, und es treten außerdem keine elektrischen Isolationsprobleme auf. Es kann ein beliebig ausgebildetes handelsübliches Ultraschall-Abtastsystem verwendet werden, das bei Bedarf rasch und einfach austauschbar ist und gegebenenfalls an anderer Stelle eingesetzt werden kann. Die spezielle Plazierung im weiteren Behälter und die Möglichkeit der Durchführung einer 360°-Drehung zusammen mit dem weiteren
Behälter oder einer anderen Halteeinrichtung führen
dazu, daß mit einer einzigen Volldrehung das gesamte
Körperteil, z.B. die weibliche Brust oder der Hoden,
in beliebig vielen Schnitten abgetastet werden kann.

Vorteilhafte Ausgestaltungen der Erfindung ergeben
sich aus den Unteransprüchen.

Von besonderem Vorteil ist dabei eine Ausführungsform,
bei der das zu untersuchende Körperteil in die Flüssigkeit des Flüssigkeitsbehälters hineinragt und bei der
das gesamte System rotiert, und zwar ohne daß eine die
Ankopplung des Ultraschall-Abtastsystems an den Flüssigkeitsbehälter störende Relativbewegung zwischen diesen
beiden Bauelementen notwendig wäre. Bei Anwendung einer
Membran ergibt sich außerdem noch die Möglichkeit, daß
das zu untersuchende Körperteil nur noch indirekt über
die Membran, die über die Durchtrittsöffnung des Auflagebrettes gespannt ist, in die Flüssigkeit des Flüssigkeitsbehälters eintaucht. Dies hat den Vorteil, daß das
eintauchende Körperteil mit der Flüssigkeit direkt nicht
in Verbindung tritt und daß dennoch eine reibungslose
Rotation des Gesamtsystems aus Flüssigkeitsbehälter
samt Halteeinrichtung mit dem daran befestigten Ultraschall-Abtastsystem möglich ist.

Weitere Vorteile und Einzelheiten der Erfindung ergeben
sich aus der nachfolgenden Beschreibung von Ausführungsbeispielen anhand der Zeichnung und in Verbindung mit den
Patentansprüchen.

Es zeigen:

Figur 1 ein erstes, bevorzugtes Ausführungsbeispiel der Erfindung in der Anwendung für Ultraschall-Mammasonographie (oder Hoden-Sonographie), teilweise im Schnitt,

Figur 2 eine um 90° gedrehte Ansicht der im Figur 1 verwendeten trichterförmigen Haltevorrichtung,

Figur 3 eine Draufsicht auf die in Figur 1 verwendete trichterförmige Haltevorrichtung,

Figur 4 ein zweites Ausführungsbeispiel der Erfindung mit einem außen auf einer feststehenden Membran umlaufenden Ultraschallkopf,

Figur 5 ein drittes Ausführungsbeispiel der Erfindung,

Figur 6 ein viertes Ausführungsbeispiel der Erfindung, bei der der Flüssigkeitsbehälter aus einem zylindrischen Körper mit schräger Trennwand gebildet ist,

Figur 7 eine Zusatzeinrichtung nach Figur 6 im Blick von oben,

Figur 8 ein fünftes Ausführungsbeispiel mit Flüssigkeits-Vorlaufstrecke, und

Figur 9 eine Modifikation der Flüssigkeits-Vorlaufstrecke nach Figur 8.

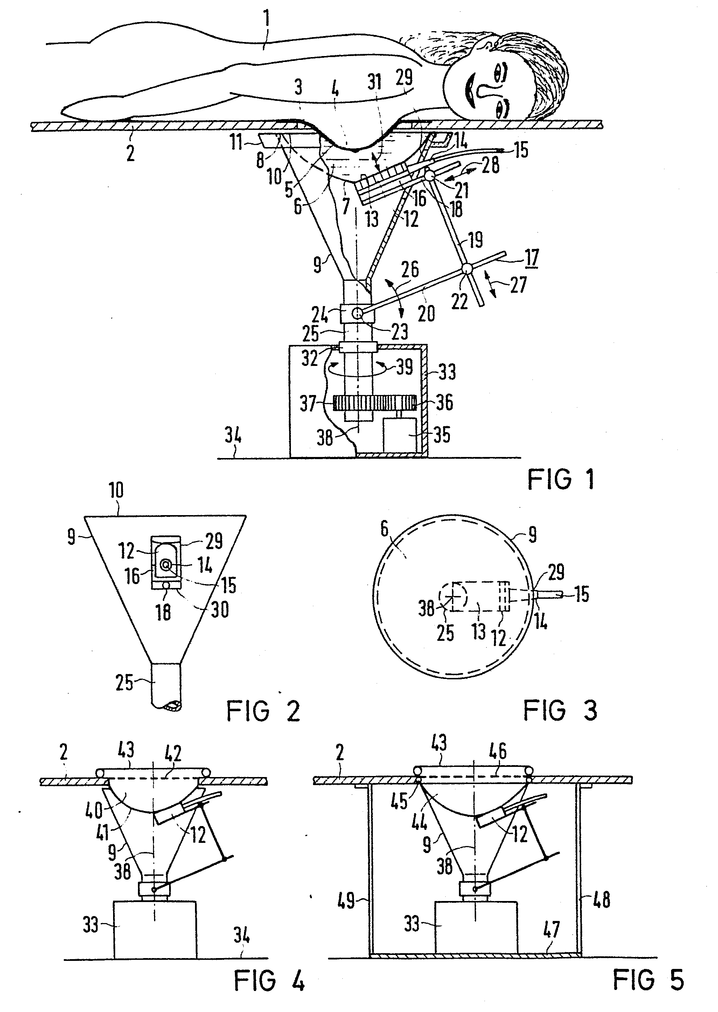

In der Figur 1 liegt eine Patientin 1 auf einem Auflagebrett 2, das Bestandteil einer Patientenliege ist. Das Auflagebrett 2 enthält eine Durchtrittsöffnung 3, über die eine Membran 5 gespannt ist. Die Patientin 1 liegt auf dem Auflagebrett 2 so, daß die zu untersuchende Brust 4 auf der Membran 5 der Durchtrittsöffnung 3 plaziert ist. Durch das Eigengewicht der
Brust 4 wird die Membran 5 in der dargestellten
Weise durch die Durchtrittsöffnung 3 des Auflagebrettes 2 hindurchgedrückt. Die Membran 5 liegt
dabei, durch ein Kontaktmittel, wie z.B. ein Gel
oder Wasser, akustisch angekoppelt, an der Oberfläche
der Brust 4 an.

Vom Flexibilitätsgrad der Membran 5 hängt es ab, wie
weit die Brust 4 durch die Durchtrittsöffnung 3 des
Auflagebrettes 2 hindurchragt. Je nach Untersuchungsfall können mehr oder weniger flexible Membranen eingesetzt werden. Speziell für die Tumoruntersuchung
empfiehlt es sich, als Membran eine Gummifolie zu
verwenden, die die Brust in einem vorgegebenen Maße
komprimiert. Auch eine Plastikfolie ist verwendbar,
wobei sich aufgrund der geringeren Elastizität eine
stärkere Kompression der Brust 4 ergibt. Ebenso läßt
sich ein textiles Gewebe, z.B. das Gewebe eines Nylonstrumpfes oder z.B. Mull einsetzen. Durch den Durchtritt von Flüssigkeit aus dem darunter befindlichen
Flüssigkeitsbad ist in diesem Fall eine akustische
Ankopplung gegeben. In der komprimierten Brust können
Tumore besser differenziert werden als in der Brust 4
bei freier Immersion. Unterschiedliche Flexibilitätsgrade können dadurch erreicht werden, daß austauschbare Membranen aus unterschiedlich flexiblen Materialien
verwendet werden. Sie können jedoch auch erreicht werden,
wenn für die Membran immer ein und dasselbe Material verwendet wird. Dann muß allerdings ein geeigneter Spannmechanismus für die Membran 5 vorgesehen sein. Mit
dessen Hilfe kann die Membran 5 in unterschiedlichem
Maße so gespannt werden, daß Untersuchungen im völlig entspannten Zustand der Brust bei freier Immersion
bis hin zu Untersuchungen mit leichter oder stärkerer
Kompression der Brust möglich sind.

Im Falle der Anwendung der gezeigten Zusatzeinrichtung
auf Hoden-Sonographie erlaubt die Spannvorrichtung bei
völliger Lockerung oder Entfernung der Membran 5 die
Untersuchung des Hodens in freier Immersion.

Im Ausführungsbeispiel der Figur 1 ist das eigentliche
System zur Untersuchung der Brust 4 mit Ultraschall
unterhalb der Durchtrittsöffnung 3 für die Brust 4 im
Auflagebrett 2 angeordnet. Das System umfaßt dabei einen
Wassersack 6, dessen flexible Sackwand oder Haut auf
der äußeren oberen Kante 8 einer Haltevorrichtung 9, z.B.
in Form eines Trichters 9, befestigt ist in der Weise,
daß der Trichter 9 den Wassersack 6 umgibt. Die Haut 7
besteht aus einer dünnen Folie oder halbkugelförmigen
Membran aus einem Ultraschall gut leitenen oder zumindest
Ultraschall durchlässigen Material, z.B. aus einem
Plastikmaterial oder Kunststoff wie Polyvenylchlorid
(PVC) oder aus Gummi. Für Versuchszwecke wurde eine
käuflich erhältliche Kunststoff-Duschhaube verwendet.
Der Wassersack 6 und die trichterförmige Haltevorrichtung 9 sind oben offen. Der Wassersack 6 ist mit Wasser
gefüllt so weit, daß bei eingetauchter Brust 4 das
Wasser bis zur Sack- bzw. Trichteröffnung 10 reicht.
Überlaufendes Wasser kann in einem Auffangbehälter,
z.B. nach Art einer Auffangrinne 11 oder eines Überlaufgefäßes, gesammelt werden. Die Behälteröffnung 10
ist in einem vorgegebenen, sehr geringen Abstand von
z.B. 2 mm von der Unterfläche des Auflagebrettes 2
zentral unter der Öffnung 3 angeordnet.

Zur Abtastung der Brust 4 dient ein Ultraschallkopf 12, und zwar bevorzugt ein Multielement-Schallkopf und speziell ein Linear-Array, mit einer Ultraschall-Applikationsfläche 13 und einer Anschlußtülle 14 für das Signalkabel 15. Der Schallkopf 15 ist Bestandteil eines handelsüblichen sonographischen Untersuchungsgeräts mit Echtzeit-Darstellung, z.B. Bestandteil des SIEMENS-Gerätes "SONOLINE 8000". Prinzipiell könnte auch ein Ultraschallkopf 12 vom bekannten Typ des mechanischen Scanners verwendet werden, z.B. ein heute gebräuchlicher Sector-Scanner. Sie Lage der einzelnen langgestreckten Ultraschallelemente eines Arrays ist in Figur 3 eingezeichnet. Das Ultraschall-Array 12 hat z.B. eine Breite von 10 cm und eine Ultraschall-Frequenz von 3,5 MHz. Es liegt in einer U-förmigen Trägerschiene 16, die an einem Tragearmgestell 17 gehaltert ist. Das Tragearmgestell 17 besteht aus drei Armteilen 18, 19 und 20. Die Armteile 18 und 19 sind über ein erstes Kreuzgelenk 21 und die Tragarme 19 und 20 über ein zweites Kreuzgelenk 22 miteinander verbunden. Das unterste Ende des Armteiles 20 ist mittels Drehgelenk 23 an einer Muffe 24 befestigt. Diese Muffe 24 sitzt wiederum am Schaft einer Drehwelle 25, die unterer Bestandteil der trichterförmigen Haltevorrichtung 9 ist. Mit Hilfe des Drehgelenkes 23 läßt sich das gesamte Haltearmgestell 17 in Richtung des Doppelpfeiles 26 schwenken. Das Kreuzgelenk 22 ist so gestaltet, daß sich das Armteil 19 in Richtung des Doppelpfeiles 27 bewegen läßt. Entsprechend läßt sich mit Hilfe des Kreuzgelenkes 21 das Armteil 18 mit der U-förmigen Trägerschiene 16 für das Ultraschall-Array 12 in Richtung des Doppelpfeiles 28 bewegen.

Wie in der Figur 2 dargestellt ist, läßt sich die U-förmige Trägerschiene 16 für das Ultraschall-Array 12 durch eine Öffnung oder einen Schlitz 29 in der Wand des Trichters 9 in das Innere des Trichters 9 hineinführen. Das Armteil 18 liegt dabei auf der unteren Kante 30 des Schlitzes 29 beweglich auf. Je nach Einstellung des Haltearmes 17 läßt sich also über den Schlitz 29 die U-förmige Trägerschiene 16 zusammen mit dem eingelegten Ultraschall-Array 12 in eine gewünschte Ankoppelposition am Wassersack 6 in den Trichter 9 einbringen. Im vorliegenden Ausführungsbeispiel ist insbesondere die dargestellte Ankoppelposition des Ultraschall-Arrays 12 von Interesse. Bei dieser Ankoppelposition liegt das Ultraschall-Array 12 mit seiner Applikationsfläche 13 unter einem vorgegebenen Winkel, d.h. mehr oder weniger geneigt, so am Wassersack 6 an, daß das Ultraschall-Array 12 senkrecht zur Oberfläche der komprimierten Brust 4 Ultraschallsignale aussendet und Echosignale empfängt. Bei Rotation wird dadurch die Kegelform der Brust 4 nachgebildet. Es ergibt sich somit eine kegelförmige Abtastbewegung der Applikationsfläche 13, was für ein optimales axiales und besonders laterales Auflösungsvermögen wichtig ist. Der Vorgang der Senkrechtbeschallung ist in der Figur 1 mit dem Doppelpfeil 31 angedeutet.

Nach Figur 1 ist die Drehwelle 25 der trichterförmigen Einrichtung 9 mit Hilfe eines Drehlagers 32 auf der Oberfläche eines Gehäuses 33 gelagert. Das Gehäuse 33 steht z.B. auf dem Fußboden 34 unterhalb des Auflagebrettes 2 der Patientenliege. Anstelle auf dem Fußboden 34 kann jedoch das Gehäuse 33 auch auf einem Zwischenbrett 47 gehaltert sein, das z.B. wiederum an dem Auflagebrett 2 der Patientenliege befestigt ist. Diese Ausführungsmöglichkeit ist z.B. in der Figur 5 angedeutet.

Im Gehäuse 33 ist ein Elektromotor 35 angeordnet. Die Drehwelle des Motors 35 greift über ein Zahnrad 36 in ein Zahnrad 37 am unteren Ende der Drehwelle 25 der Halteeinrichtung 9. Sobald der Motor 35 eingeschaltet ist, wird über das Zahnradgetriebe 36, 37 und die Drehwelle 25 die Halteeinrichtung 9 zusammen mit dem Wassersack 6 und dem am Wassersack 6 angekoppelten Ultraschall-Array 12 um eine zentrale Drehachse 38 kontinuierlich gedreht oder geschwenkt. Der Schwenkvorgang erfolgt dabei um 360° in der einen Drehrichtung und dann wieder um 360° entgegen der vorherigen Drehrichtung zurück in die Ausgangsposition. Dieser Vorgang ist durch den Doppelpfeil 39 angedeutet. Während das System dreht, tastet das Ultraschall-Array 12 die Brust 4 in einer Vielzahl von Querschnitten mit Ultraschall nach dem Impuls-Echo-Verfahren ab. Die aus den verschiedenen Schnittebenen der Brust 4 anfallenden Echosignale werden in üblicher Weise an einem (nicht dargestellten) Bildgerät, insbesondere mit Kathodenstrahlröhre, an dem das Ultraschall-Array 12 über das Signalkabel 15 angeschlossen ist, in Form von sich kontinuierlich ändernden Echoschnittbildern aufgezeichnet. Die kontinuierliche Bildänderung erscheint für eine Beurteilung günstiger als eine Abfolge stehender Schnittbilder.

Mit der in der Figur 1 dargestellten Abtastvorrichtung läßt sich also mit einer einzigen 360°-Drehung die weibliche Brust in einer Vielzahl von Schnitten abtasten. Es rotiert das gesamte System aus Wassersack 6 und Halteeinrichtung 9 zusammen mit dem im Trichter 9 befindlichen Ultraschall-Array 12. Wegen der Neigung des Arrays 12 zur Rotationsachse 38 ergibt sich eine Bewegung des Ultraschallkopfes auf einem kegelmantelförmigen Abtastweg. Zwischen dem Wasser des Wassersackes 6 und der über die Membran 5 angekoppelten Brust 4 treten praktisch keine Reibungsprobleme auf. Da sich das Ultraschall-Array 12 außerhalb des Wassers des Wassersackes 6 befindet, braucht das Ultraschall-Abtastsystem nicht extra abgedichtet zu werden, und es treten hinsichtlich des Signalkabelanschlusses keine elektrischen Isolationsprobleme auf.

Das Ultraschall-Array 12 ist, wie bereits erwähnt, von handelsüblicher Art. Dies hat den besonderen Vorteil, daß es jederzeit ausgewechselt und bei Bedarf für andere Anwendungsfälle oder Aufgabenbereiche eingesetzt werden kann. Die Auflage 2, der Wassersack 6, die Halteeinrichtung 9 und der Antrieb des Ultraschallkopfes 12 sind als Zusatzgerät zum Ultraschallkopf 12 zu betrachten. D.h. ein bereits vorhandener Ultraschallkopf 12 kann durch dieses Zusatzgerät für die Anwendung in der Mammasonographie nachgerüstet werden.

Die Figur 3 zeigt eine Draufsicht auf die Abtastvorrichtung. Die eingezeichneten Bezugsziffern stimmen für die dargestellten Bauelemente mit jenen der Figur 1 überein.

Die Figur 4 zeigt eine weitere Lösung dahingehend, daß ein Wassersack 40 mit seiner Haut 41 jetzt nicht mehr an der Oberkante der trichterförmigen Halteeinrichtung 9, sondern in der Öffnung 3 des Auflagebrettes 2 befestigt ist. Damit ist also der Wassersack 40 jetzt feststehend. Die trichterförmige Halteeinrichtung 9 könnte hier völlig entfallen. Vorliegend ist sie lediglich eine rotierende Schutzeinrichtung. Entsprechend dreht sich auch das Ultraschall-Array 12 an der Ankoppelstelle relativ zum feststehenden Wassersack 40 um die Drehachse 38.

Bei einer Ausführungsform wie die der Figur 4, wo also der Wassersack 40 feststeht, empfiehlt es sich, als Membran 42 zur Ankopplung der Brust an das Wasser des Wassersackes 40 eine perforierte oder sonstwie mit Aussparungen versehene Membran 42 zu verwenden. Die Perforation bzw. die sonstigen Aussparungen lassen es zu, daß einerseits beliebig Wasser in den Wassersack 40 gefüllt und andererseits von der Brust beim Eintauchen zusammen mit der Membran 42 in den feststehenden Wassersack 40 gegebenenfalls verdrängtes Wasser durch die Perforation oder·die Aussparungen abfließen kann. Um das überfließende Wasser zu sammeln, liegt vorzugsweise auf dem Auflagebrett 2 konzentrisch zur Durchtrittsöffnung 3 ein schmaler, aufblasbarer Gummiring 43. Im Inneren dieses Ringes 43 sammelt sich das überlaufende Wasser, wenn die Patientin die zu untersuchende Brust 4 zusammen mit der Anlagemembran 42 durch den Ring 43 hindurch in das Wasser des Wassersackes 40 eintaucht. Der aufblasbare Gummiring 43 gestattet außerdem eine besonders weiche Lagerung der Brustpartie. Ein solcher Ring 43 kann selbstverständlich auch bei der Vorrichtung der Figur 1 eingesetzt werden. Ebenso kann auch die Membran 5 in Figur 1 bei Bedarf perforiert sein. Der Ring 43 dient dann gleichzeitig wieder als Auffangbehälter für überfließendes Wasser. Ist die Membran 5 hingegen nicht perforiert, d.h. wasserundurchlässig, so sollte vor Inbetriebnahme der Wassersack 6 nur so weit mit Wasser gefüllt sein, daß beim Eintauchen der Brust nur wenig oder überhaupt kein Wasser überläuft.

Die Figur 5 zeigt eine weitere Lösung. Hier ist ein Wassersack 44 wieder mit einer trichterförmigen Halteeinrichtung 9 fest verbunden. Der Trichter 9 ist jedoch an seiner Oberkante mittels eines z.B. als ringförmiges Kugellager ausgebildeten Drehlagers 45 in der Durchtrittsöffnung 3 oder unterhalb dieser am Auflagebrett 2 drehbar gehaltert. In diesem Ausführungsbeispiel werden also Trichter 9 und Wassersack 44 zusammen mit dem angekoppelten Ultraschall-Array 12 wieder gemeinsam um die Drehachse 38 gedreht. Auch hier kann die Membran 46 wieder perforiert sein, und ein Ring 43 kann wieder als Auffangbehälter für überlaufendes Wasser dienen. Was als wichtig angesehen wird: Hier steht die gesamte Abtastvorrichtung auf einem Zwischenbrett 47, das mittels Haltestangen 48, 49 od. dgl. am Brett 2 der Patientenliege befestigt ist.

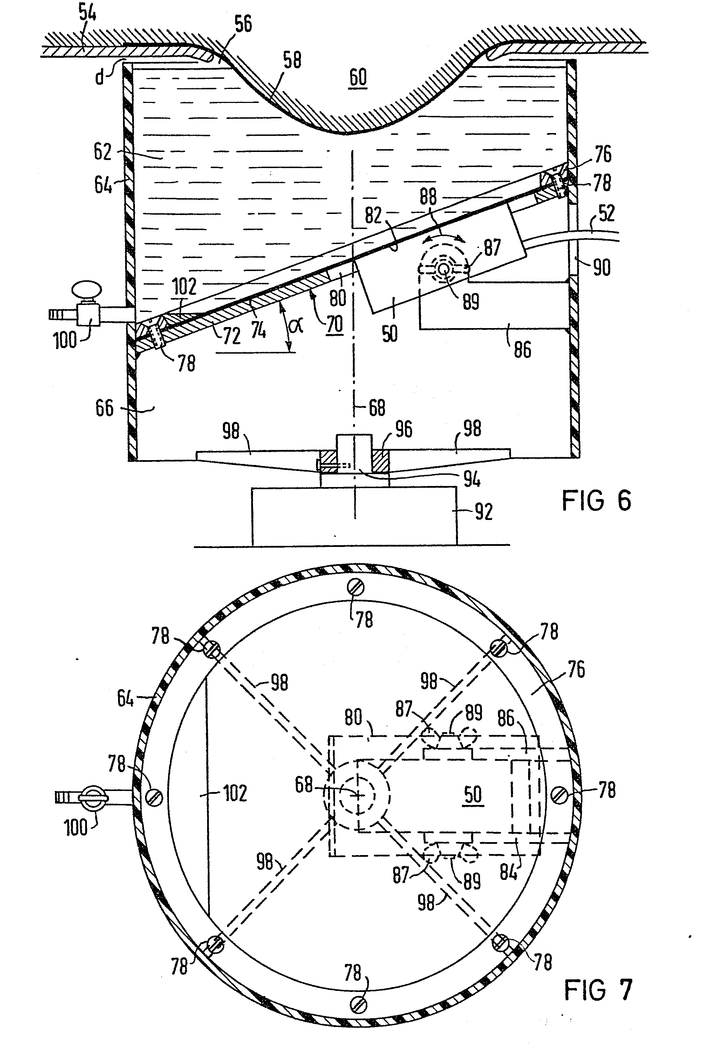

In den Figuren 6 und 7 ist wiederum eine Zusatzeinrichtung zu einem handelsüblichen Echtzeit-Ultraschall-Untersuchungsgerät dargestellt. Das Echtzeit-Ultraschall-Untersuchungsgerät selbst ist wieder an sich bekannt und mit Ausnahme des zugehörigen Abtastkopfes 50 samt Verbindungskabel 52 nicht gezeigt. Die Zusatzeinrichtung ist hier wieder insbesondere für Routineuntersuchungen der weiblichen Brust vorgesehen.

Die Patientin liegt auf einem Patienten-Auflagebrett 54, welches mit einer Durchtrittsöffnung 56 versehen ist. Die Durchtrittsöffnung 56 ist von einer flexiblen
Membran 58, die insbesondere aus einer Gummihaut bestehen kann, überspannt. Die Brust 60 der Patientin
liegt an der Oberfläche der Membran 58 eng an und wird
durch diese zu einem gewissen Grade komprimiert. Es ist
festzuhalten, daß dabei die Membran 58 die Form der
komprimierten Brust 60 annimmt. Zwischen der Membran 58
und der Brust 60 kann ein Kontakt- oder Ankoppelmedium
wie beispielsweise ein Gel vorhanden sein.

Bei der Untersuchung taucht die Membran 58 von oben in
ein Flüssigkeitsbad ein, das sich in einem oberen Behälter 62 befindet. Als Ultraschall gut leitende
Flüssigkeit wird dabei bevorzugt Wasser verwendet.
Der obere Behälter 62 wird durch einen zylindrischen
Körper oder Zylinder 64 gebildet, der auch einen unteren
Raum oder Behälter 66 beinhaltet. Der zylindrische
Körper 64 ist in einem verhältnismäßig geringen Abstand d von der unteren Fläche der Patientenauflage
54 angeordnet. Der Zylinder 64 ist relativ breit und
flach ausgebildet. Er kann insbesondere aus einem
Kunststoff bestehen. Wie später näher ausgeführt ist,
rotiert der zylindrische Körper 64 im Untersuchungsbetrieb um seine Zylinder- oder Längsachse 68, die bei
der Untersuchung vertikal ausgerichtet ist.

In den Zylinder 64 ist eine schräge, flüssigkeitsdichte
Trennwand 70 eingezogen. Diese Trennwand 70 trennt den
Zylinderinnenraum in den oberen und den unteren Behälter 62 bzw. 66. Bei dieser Konstruktion kann man
davon sprechen, daß der untere Behälter 66 den oberen
Behälter 62 "trägt". Die Trennwand 70 umfaßt einen
Boden 72 aus einem verhältnismäßig stabilen Material
(z.B. Kunststoff oder Metall), der auf seiner Oberseite eine flexible Folie oder Membran 74 trägt. Diese Membran 74, die bevorzugt aus Gummi bestehen kann, wird am Innenrand des Zylinders 64 durch einen elliptischen Haltering 76 flüssigkeitsdicht festgehalten. Dieser Haltering 76 ist durch geeignete Haltemittel, wie beispielsweise Verschraubungen 78, an den Rand der Membran 74 gepreßt. Stattdessen kann auch eine Klebeverbindung zwischen der Membran 74 und dem Boden 72 gewählt sein. Der Boden 72 kann, wie erwähnt, aus Kunststoff bestehen. Er kann beispielsweise in den Zylinder 64 eingeklebt oder eingeschweißt sein.

Der Boden 72 und damit auch die Membran 74 sind gegenüber der Horizontalen um einen Winkel $\alpha$ geneigt. Dieser Winkel $\alpha$ kann, wie Versuche ergeben haben, für viele Anwendungsfälle einheitlich etwa 20° betragen. Der Neigungswinkel zwischen Zylinderachse 68 und Trennwand 70 beträgt somit ungefähr $(90° - \alpha) = 70°$.

Der Boden 72 besitzt eine rechteckige Ausnehmung 80, in deren Mitte der Ultraschallkopf 50 angeordnet ist. Hierbei kann es sich insbesondere um einen herkömmlichen Ultraschallkopf 50 mit etwa rechteckiger Stirnseite oder Abstrahl- und Empfangsfläche 82 für Echtzeit-Bildstellung nach dem Puls-Echo-Verfahren handeln. Bevorzugt wird also ein lineares Array von Ultraschall-Wandlerelementen konventioneller Bauart verwendet. Aus Figur 7 ergibt sich, daß zwischen den Kanten der etwa rechteckigen Abstrahlfläche 82 und den Kanten der rechteckigen Ausnehmung 80 jeweils ein definierter Abstand vorgesehen ist.

Die Abstrahlfläche 82 des Ultraschallkopfes 50 ist gegenüber der Horizontalen ungefähr um den Winkel $\alpha$ geneigt. Sie liegt dazu im unteren Behälter 66 an der Unterseite der Membran 74 an. Der Ultraschallkopf 50 wird dabei durch zwei Winkelarme 84 und 86 getragen, die mit der Innenwand des Zylinders 64 im unteren Behälter 66 endseitig starr verbunden sind. Dabei kann der Ultraschallkopf von Hand mittels Flügelschrauben 87 gelöst und um eine horizontale Achse 89 gekippt oder geschwenkt werden, was auch durch einen Doppelpfeil 88 angedeutet ist. Durch Kippen des Ultraschallkopfes 50 um diese Achse 89 wird erreicht, daß die an der Membran 74 anliegende Abstrahlfläche 82 parallel zur Hautoberfläche der Brust 60 eingestellt werden kann. Der Kippbereich $\Delta\alpha$ braucht dabei nur verhältnismäßig gering zu sein und kann beispielsweise nur wenige Grad betragen, da die Grobeinstellung bereits schon weitgehend durch die erwähnte Anordnung des Bodens 72 unter dem Winkel ($90^{o} - \alpha$) bezüglich der Vertikalen (Zylinderachse 68) erreicht ist. Beim Kippen aus der gezeigten Winkellage $\alpha$ heraus wird die Gummimembran 74 leicht an einer Seite gedehnt.

Wie bereits erwähnt, kann zum Festhalten und Kippen um die horizontale Achse 89 ein lösbares Befestigungsmittel wie z.B. eine Schraubverbindung mit Flügelschrauben 87 vorgesehen sein. Mittels dieser Schraubverbindung ist der Ultraschallkopf 50 fest, aber jederzeit lösbar mit den Winkelarmen 84, 86 und damit mit dem Zylinder 64 verbunden. Nach Abschrauben von den Haltearmen 84, 86 kann daher der Ultraschallkopf 50 auch für andere Zwecke als die Mamma-Sonographie verwendet werden. Hierzu werden der Ultraschallkopf 50 und das Zuleitungskabel 52 durch eine Öffnung 90 in der unteren Zylinderwand herausgeführt.

Zum kontinuierlichen Antrieb des Zylinders 64 ist ein Antriebsmotor 92 vorgesehen. Die Rotation um die Zylinderachse 68 kann sich dabei über einen Winkelbereich von z.B. $\pm$ 360$^\circ$ erstrecken. Die Antriebsachse
94 des Antriebsmotors 92 ist koaxial zur Zylinderachse 68 ausgerichtet und fällt mit der Vertikalen
zusammen. Die Zylinderachse 68 verläuft dabei etwa
in der Mitte der Durchtrittsöffnung 56. Der Zylinder
64 ist mittels eines Aufsetzringes 96 und mit Hilfe
von vier sternförmig hiervon ausgehenden Haltestangen
98 an der Antriebsachse 94 befestigt.

Bei einer Untersuchung der Patientin wird der Antriebsmotor 92 im Vorlauf (bis + 360$^\circ$) und Rücklauf
(bis - 360$^\circ$) betrieben. Dabei liegt die Abstrahlfläche 82 des Ultraschallkopfes 50 innerhalb des
unteren Behälters 66 an der flexiblen Trennwand 74
unter Ultraschallankopplung an. Die Abstrahlfläche
82 ist hierbei auf den oberen Flüssigkeitsbehälter
62 und insbesondere auf die zu untersuchende Brust
60 gerichtet. Bei Betrieb des Antriebsmotors 92 dreht
sich der Zylinder 64 unterhalb der Brust 60 kontinuierlich um die vertikale Achse 68, und die Abstrahl- und
Empfangsfläche 82 des Ultraschallabtastkopfes beschreibt
dabei einen kegelmantelförmigen Abtastweg. Das zu untersuchende Organ 60 wird somit aus jeder Winkelposition
ultraschallmäßig abgetastet.

An der tiefsten Stelle des oberen Behälters 62 befindet
sich ein Wassereinlauf und -ablauf 100 in Form einer
verschließbaren Tülle. Zur Ableitung der Ultraschall
gut leitenden Flüssigkeit in Richtung auf den Ein- und
Ablauf 100 ist ein Führungsstück 102 benachbart zum
Ring 76 angeordnet.

Die in den Figuren    und    gezeigte Ausführungsform
einer Zusatzeinrichtung zu einem handelsüblichen Echtzeit-Ultraschall-Untersuchungsgerät besitzt einige besonders erwähnenswerte Vorteile. Bei dieser Ausführungsform ist nur ein einziger Zylinder 64 vorgesehen, um das Flüssigkeitsgefäß 62 einerseits und den Schutzraum für den Ultraschallkopf 50 sowie für dessen Haltevorrichtung 84, 86, 87 zu bilden. Der Ultraschallkopf 50 befindet sich außerhalb des Wasserbades, so daß er von den Seitenarmen 84, 86 leicht abgenommen, aus der Austrittsöffnung 90 herausgeführt und für andere Untersuchungszwecke verwendet werden kann. Als weiterer Vorteil ist es anzusehen, daß die Ultraschall-Ankopplung auf den Bereich des Ultraschallkopfes 50 beschränkt ist. Dieses liegt an der rechteckigen Ausnehmung 80 vorgegebener Größe im Boden 72, die auf die Größe der Abstrahlfläche 82 abgestimmt ist. Ein weiterer Vorteil besteht darin, daß der Ein- und Ablauf 100 für die Flüssigkeit an der tiefsten Stelle des Flüssigkeitsbehälters 62 angeordnet werden kann. Ein Vorteil besteht nicht zuletzt auch darin, daß sich das Zusatzgerät auf recht. einfache Weise konstruktiv realisieren läßt.

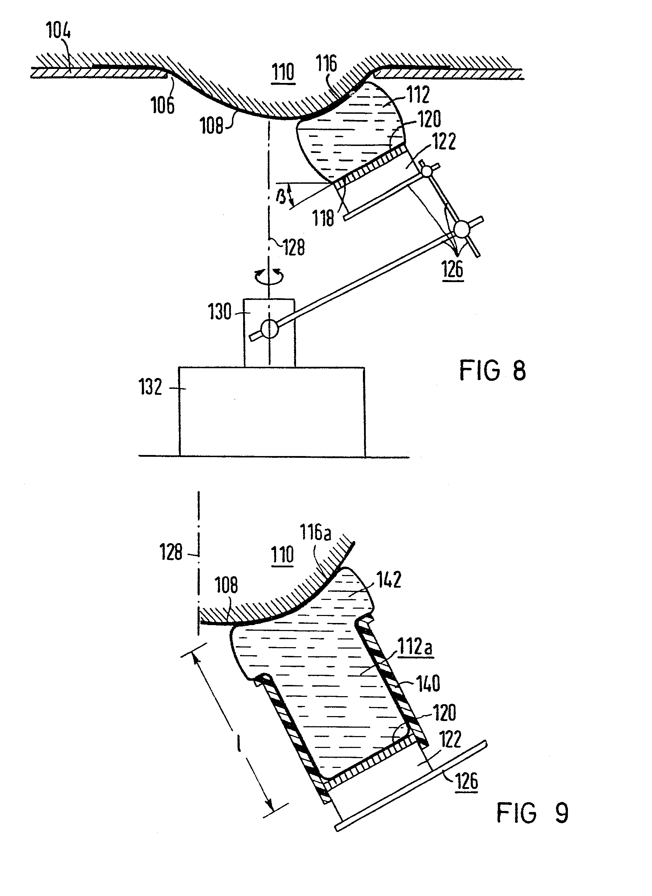

In Figur 8 ist eine weitere Zusatzeinrichtung zu einem handelsüblichen Echtzeit-Ultraschall-Untersuchungsgerät (nicht gezeigt) dargestellt. Diese Ausführungsform ist insbesondere für Mamma-Untersuchungen vorgesehen.

Nach Figur 8 ist wiederum eine Patientenliege 104 mit einer Durchtrittsöffnung 106 vorgesehen. Auch in diesem Fall ist die Öffnung 106 von einer Membran 108 abgedeckt. Die Membran 108 besteht wiederum bevorzugt aus einer Gummifolie. Bei dieser Ausführungsform liegt die komprimierte Brust 110 der Patientin unter Zwischenlage eines Kontaktgels ebenfalls direkt an der Membran 108 an. Die Membran 108 unterteilt den Raum wiederum in einen patientenseitigen Raumteil und in einen apparaturseitigen Raumteil.

Auf der apparaturseitigen Seite der Membran 108 ist eine Flüssigkeits-Vorlaufstrecke 112 vorgesehen. Diese Flüssigkeits-Vorlaufstrecke 112 ist ein allseitig geschlossener Beutel oder Sack aus einem flexiblen Material, der mit einer geeigneten, Ultraschall gut leitenden Flüssigkeit wie beispielsweise Wasser gefüllt ist. Der Beutel 112 besteht aus einer dehnbaren Haut, z.B. aus einer Gummihaut oder einer anderen flexiblen Folie. Er besitzt eine flexible Vorderfläche 116, die als Gleitfläche ausgebildet ist, und eine dieser gegenüberliegende Rückfläche 118, die als Auflagefläche verwendet wird. Die Flächengröße von Vorder- und Rückseite 116 bzw. 118 entspricht im wesentlichen der Abstrahlfläche 120 des Ultraschall-Applikators oder Ultraschallkopfes 122. Die Auflagefläche 118 ist an die Abstrahlfläche 120 ultraschalldurchlässig angekoppelt und dort lösbar befestigt. Die flexible Gleitfläche 116 dagegen ist an die Membranoberfläche zwar ebenfalls ultraschallmäßig angekoppelt, doch lediglich angelegt. Aus Figur 8 wird deutlich, daß der Sack 112 nicht prall mit Flüssigkeit gefüllt ist. Es handelt sich vielmehr um eine mäßige Füllung derart, daß sich die Gleitfläche 116 bei an der Membran 108 anliegender Brust 110 der Form der Membran 108 und damit auch der Kontur der Brust 110 anpaßt. Wie ersichtlich, hat die Gleitfläche 116 deswegen eine konvexe Form.

Zwischen der Membran 108 und der Flüssigkeits-Vorlaufstrecke 112 ist gleichfalls ein Kontaktgel oder ein anderes haftendes Medium vorgesehen. Dieses wirkt gleichzeitig als Gleitsubstanz.

Wie bereits erwähnt, ist auf der Rückseite 118 des Beutels 112, wiederum unter Zwischenschaltung eines Kontaktgels, der herkömmlich ausgebildete Ultraschallkopf 122 für Echtzeit-Untersuchungen nach dem Puls-Echo-Verfahren angeordnet. Der Ultraschallkopf 122 und der Beutel 112 sind hier lösbar miteinander verbunden. Der Ultraschallkopf 122 selbst ist an einer Haltevorrichtung 126 lösbar befestigt. Diese Haltevorrichtung 126 kann, wie in Figur 3 dargestellt, durch ein Gestänge gebildet sein, das durch verstellbare Gelenke miteinander verbunden ist. Auch anders ausgebildete Haltevorrichtungen lassen sich verwenden.

Der Ultraschallkopf 122 und seine Haltevorrichtung 126 sind um eine Rotationsachse 128, die bevorzugt vertikal angeordnet ist, drehbar. Dazu ist die Haltevorrichtung 126 an der Welle 130 eines Antriebsmotors 132 befestigt. Auch hier ist bedeutsam, daß die Abstrahlfläche 120 nicht parallel, sondern schräg zur Rotationsachse 128 und hier speziell auch zur Horizontalen ausgerichtet ist. Der einstellbare Winkel zwischen der Horizontalen und der Abstrahlfläche 120 ist hier mit $\beta$ bezeichnet. Dieser Neigungswinkel $\beta$ kann bevorzugt etwa $20^{\circ}$ betragen. Auch hier soll wieder betont werden, daß sich mit einer solchen Einstellung viele Anwendungsfälle erfassen lassen.

Auch in diesem Fall wird der Antriebsmotor 132 eine kontinuierliche Drehung erzeugen, wobei ein Drehbereich von $\pm 180^{\circ}$ oder aber $\pm 360^{\circ}$ vorgegeben sein kann. Beim Drehen der Welle 130 gleitet die Vorderseite oder Gleitfläche 116 des Beutels 112 an der Außenseite der Membran 108 entlang, und der Ultraschallkopf 122 tastet dabei im Echtzeit-Verfahren die Brust 110 mit Ultraschall ab.

Bei der in Figur 8 dargestellten Ausführungsform wird eine Flüssigkeits-Vorlaufstrecke 112 von vergleichsweise geringem Flüssigkeitsvolumen benötigt. Das Flüssigkeitsvolumen ist dabei allseitig abgeschlossen, so daß hier nichts überlaufen kann. Die Ankopplung mit Ultraschall ist bei dieser Ausführungsform im wesentlichen auf den Bereich des Ultraschallkopfes 122 beschränkt. Auch hier ist nach entsprechender Einstellung die Abstrahlfläche 120 des Ultraschallkopfes 122 im wesentlichen parallel zur abgetasteten Brust-Oberfläche ausgerichtet.

Es soll noch einmal hervorgehoben werden, daß die Vorlaufstrecke 112 nur mäßig mit der Ultraschall gut leitenden Flüssigkeit gefüllt und dabei so ausgebildet ist, daß die flexible Gleitfläche 116 in Anpassung an die Brust 110 eine konvexe Form erhält. Ein Ausführungsbeispiel, wie dieses erreicht werden kann, wobei gleichzeitig eine stabile Befestigung sichergestellt ist, wird in Figur 9 gezeigt.

Nach Figur 9 ist die Flüssigkeits-Vorlaufstrecke 112a in einer Halterung 140 untergebracht. Diese Halterung 140 ist im wesentlichen eine der Kopfform angepaßte Hülse, in der sich der größte Teil des Flüssigkeitssackes 112a befindet. Letzterer besitzt ein pilzförmiges Aussehen infolge eines vergrößerten Vorderoder Kopfteils 142, das aufgrund des Andruckes an der Brust 110 die verbreiterte Form erhält. Die flexible Gleitfläche 116a ist dabei natürlich von der Halterung 140 freigelassen. Auch hier ergibt sich wieder eine konkave Form der Vorderfläche 116a. Diese Vorderlfäche 116a gleitet auch hier wieder im Betrieb über die die Brust 110 komprimierende Membran 108 hinweg. Die Halterung 140 kann aus einem beliebigen Material, beispielsweise aus einem Kunststoff wie Plexiglas bestehen. Bevorzugt sollte die Halterung 140 am Ultraschallkopf 122 lösbar befestigt sein. Dieses kann dadurch erreicht werden, daß der hintere Teil der Hülse 140 das Vorderteil des Ultraschallkopfes 122 umfaßt. In diesem Fall kann der Ultraschallkopf 122 verhältnismäßig leicht von der Rückfläche 118 des Flüssigkeitssackes 112a gelöst und für andere Anwendungszwecke eingesetzt werden.

Es hat sich gezeigt, daß der Abstand 1 (siehe Figur 9) zwischen der Abstrahlfläche 120 des Ultraschallkopfes 122 und der Vorderfläche 116a des pilzförmigen Kopfteils 142 etwa 5 bis 6 cm lang sein sollte, wenn bei einer Ultraschallfrequenz von 3,5 MHz die Schichtmitte des untersuchten Gewebes der Brust 110 in der Fokuslinie des Ultraschallkopfes 122 plaziert werden soll.

Die in Figur 8 und 9 gezeigten Ausführungsformen vereinigen den Vorteil eines abgeschlossenen Flüssigkeitsvolumens mit dem Vorteil einer beschränkten Ultraschall-Ankoppelfläche. Ebenso ist bemerkenswert, daß hier ein verhältnismäßig einfacher Aufbau vorliegt. Auch hier ergibt sich wiederum, daß im Betrieb die Abstrahlfläche 120 auf einem Kegelmantel um das zu untersuchende Körperteil 110 umläuft.

32 Patentansprüche
9 Figuren

## Patentansprüche

1. Zusatzeinrichtung zu einem handelsüblichen Ultraschall-Untersuchungsgerät, das einen Ultraschallkopf zum Abtasten eines zu untersuchenden Körperteiles aufweist, mit einem Flüssigkeitsbehälter, der eine Ultraschall gut leitende Flüssigkeit enthält, mit einem Patientenauflagebrett mit Durchtrittsöffnung, durch die das zu untersuchende Körperteil hindurchragt, mit einer Haltevorrichtung, die den Ultraschallkopf festhält, und mit einer motorisch betriebenen Einrichtung zur gemeinsamen kontinuierlichen Drehung der Haltevorrichtung und des Ultraschallkopfes um eine Rotationsachse, wobei die Abstrahlfläche des Ultraschallkopfes um das zu untersuchende Körperteil umläuft, g e - k e n n z e i c h n e t   d u r c h   die Kombination der folgenden Merkmale:

a) das Ultraschall-Untersuchungsgerät ist ein Echtzeit-Ultraschall-Untersuchungsgerät,

b) am Flüssigkeitsbehälter (6; 40; 44; 64) befindet sich eine Ultraschall-Ankoppelstelle, an der der Ultraschallkopf (12) von außen anliegt,

c) die Haltevorrichtung (16, 18, 19, 20; 84, 86) hält den Ultraschallkopf (12; 50) an der Ankoppelstelle außerhalb des Flüssigkeitsbehälters (6; 40; 44; 64) lösbar fest, wobei die Abstrahlfläche des Ultraschallkopfes (12; 50) bezüglich der Rotationsachse (38; 68) schräg ausgerichtet ist, und

d) die Rotationsachse (38; 68), um die die motorisch betriebene Einrichtung (33; 92) die Haltevorrichtung und den am Flüssigkeitsbehälter von außen anliegenden Ultraschallkopf (12; 50) gemeinsam kontinuierlich dreht, weist auf die Durchtrittsöffnung (3; 56), so daß die Abstrahlfläche des Ultraschallkopfes (12; 50) auf einem Kegelmantel um das zu untersuchende Körperteil (4; 60) umläuft (Fig. 1 bis 9).

2. Zusatzeinrichtung nach Anspruch 1,   g e k e n n - z e i c h n e t   d u r c h

a) ein Patientenauflagebrett (27; 54) mit Durchtrittsöffnung (3; 56), durch die das zu untersuchende Körperteil (4) in den Flüssigkeitsbehälter (6; 44; 64) ragt,

b) eine weitere Haltevorrichtung (9; 9, 45; 94, 96, 98), die den Flüssigkeitsbehälter (6; 44; 64) im Abstand (d) vom oder drehbar am Patientenauflagebrett (2; 54) hält, und

c) eine Antriebsverbindung zwischen der motorisch angetriebenen Einrichtung (33; 92) und dem Flüssigkeitsbehälter (6; 44; 64) dergestalt, daß sich die weitere Haltevorrichtung (9; 9, 45; 94, 96, 98) und der Flüssigkeitsbehälter (6; 44; 64) mit unbewegt anliegendem Ultraschallkopf (12; 50) kontinuierlich um die Rotationsachse (38; 68) drehen, die im Raum vertikal ausgerichtet ist (Fig. 1, 2, 3, 5, 6, 7).

3. Zusatzeinrichtung nach Anspruch 1,   g e k e n n - z e i c h n e t   d u r c h

a) eine bei der Untersuchung raumfeste Anordnung des Flüssigkeitsbehälters (40),

b) ein Patientenauflagebrett (2) mit Durchtrittsöffnung (3), durch die das zu untersuchende Körperteil (4) bei der Untersuchung hindurchragt, und

c) eine Antriebsverbindung zwischen der motorisch angetriebenen Einrichtung (33) und dem Ultraschallkopf (12) zur gemeinsamen kontinuierlichen Drehung der Haltevorrichtung (16, 18, 19, 20) und des am raumfesten Flüssigkeitsbehälter (40) anliegenden Ultraschallkopfes (12) um die besagte Rotationsachse (38), wobei die Abstrahlfläche des Ultraschallkopfes (12) auf der Wand (41) des Flüssigkeitsbehälters (6) entlanggleitet (Fig. 4).

4. Zusatzeinrichtung nach einem der Ansprüche 1 bis 3, d a d u r c h   g e k e n n z e i c h n e t ,   daß das zu untersuchende Körperteil (4) über eine Membran (5; 42; 46) mit vorgebbarer Flexibilität in den Flüssigkeitsbehälter (6; 40; 44) ragt.

5. Zusatzeinrichtung nach Anspruch 4,   d a d u r c h   g e k e n n z e i c h n e t ,   daß der Flüssigkeitsbehälter (40) direkt an seiner Behälteröffnung (10) mit der Membran (42) überzogen ist.

6. Zusatzeinrichtung nach Anspruch 4,   d a d u r c h   g e k e n n z e i c h n e t ,   daß die Membran (5; 42; 46) über die Durchtrittsöffnung (3) des Patientenauflagebrettes (2) gespannt ist.

7. Zusatzeinrichtung nach einem der Ansprüche 1 bis 6, d a d u r c h   g e k e n n z e i c h n e t ,   daß der Flüssigkeitsbehälter (6; 44) und die Haltevorrichtung (16, 18 - 20) als Standgerät auf einer Standfläche (34, 47) unter der Durchtrittsöffnung (3) des Patientenauflagebrettes (2) angeordnet sind.

8. Zusatzeinrichtung nach Anspruch 7, d a d u r c h
g e k e n n z e i c h n e t , daß die Standfläche
(47) am Patientenauflagebrett (2) befestigt ist.

9. Zusatzeinrichtung nach Anspruch 7, d a d u r c h
g e k e n n z e i c h n e t , daß das sich nicht
drehende Gehäuse (33) der motorischen Einrichtung fest
mit der Standfläche (34; 47) verankert oder nach Art
eines Hängegehäuses mittels starrer oder stativartig
schwenkbarer Halterung am Patientenauflagebrett (2)
gehaltert ist.

10. Zusatzeinrichtung nach einem der Ansprüche 2 bis 9,
d a d u r c h g e k e n n z e i c h n e t , daß
die weitere Haltevorrichtung ein den Flüssigkeitsbehälter
(6; 30) einschließender weiterer Behälter (9) ist
(Figuren 1 bis 4).

11. Zusatzeinrichtung nach Anspruch 10, d a d u r c h
g e k e n n z e i c h n e t , daß die Öffnung (10)
des weiteren Behälters (9) in vorgegebenem Abstand (d)
unterhalb des Patientenauflagebrettes (2) angeordnet
ist.

12. Zusatzeinrichtung nach Anspruch 10, d a d u r c h
g e k e n n z e i c h n e t , daß der weitere Behälter
(9) mit seinem oberen Behälterrand mittels eines Drehlagers (45) in der Durchtrittsöffnung (3) des Patientenauflagebrettes (2) drehbar gelagert ist (Figur 5).

13. Zusatzeinrichtung nach einem der Ansprüche 10 bis
12, d a d u r c h g e k e n n z e i c h n e t ,
daß der weitere Behälter (9) am Boden mit einer Drehwelle (25) versehen ist, und daß zum Antrieb des weiteren
Behälters (9) ein Rotationsantrieb (35, 36, 37), der an
der Drehwelle (25) angreift, vorgesehen ist.

14. Zusatzeinrichtung nach Anspruch 13, d a d u r c h g e k e n n z e i c h n e t , daß der weitere Behälter (9) mit der Drehwelle (25) auf dem Gehäuse (33) mittels eines Drehlagers (32) drehbar gelagert ist.

15. Zusatzeinrichtung nach einem der Ansprüche 10 bis 14, d a d u r c h g e k e n n z e i c h n e t , daß der weitere Behälter (9) die Form eines Trichters hat.

16. Zusatzeinrichtung nach einem der Ansprüche 1 bis 15, d a d u r c h g e k e n n z e i c h n e t , daß der Flüssigkeitsbehälter (6; 40; 44) die Form eines Flüssigkeitssackes hat.

17. Zusatzeinrichtung nach Anspruch 16, d a d u r c h g e k e n n z e i c h n e t , daß die Mantelhaut (7) des sackförmigen Flüssigkeitsbehälters (6) aus einem flexiblen Material besteht.

18. Zusatzeinrichtung nach einem der Ansprüche 1 bis 17, d a d u r c h g e k e n n z e i c h n e t , daß der Flüssigkeitsbehälter mit Wasser gefüllt ist.

19. Zusatzeinrichtung nach einem der Ansprüche 4 bis 18, d a d u r c h g e k e n n z e i c h n e t , daß die Membran (42) mit Aussparungen, z.B. in Form von Perforationen, für den Flüssigkeitsdurchtritt oder Flüssigkeitsüberlauf versehen ist.

20. Zusatzeinrichtung nach Anspruch 19, d a d u r c h g e k e n n z e i c h n e t , daß dem weiteren Behälter (9) ein Auffangbehälter (11; 43) für überlaufende Flüssigkeit zugeordnet ist.

21. Zusatzeinrichtung nach Anspruch 20, d a d u r c h g e k e n n z e i c h n e t , daß der Auffangbehälter (11) eine um den Rand (8) des weiteren Behälters (9) laufende Rinne ist.

22. Zusatzeinrichtung nach Anspruch 20, d a d u r c h g e k e n n z e i c h n e t , daß der Auffangbehälter (43) ein aufblasbarer Ring aus flexiblem Material ist, der auf dem Patientenauflagebrett (2) konzentrisch zur Durchtrittsöffnung (3) angeordnet ist und der zugleich zur weichen Lagerung des zu untersuchenden Körperteils dient.

23. Zusatzeinrichtung nach einem der Ansprüche 10 bis 22, d a d u r c h g e k e n n z e i c h n e t , daß der Ultraschallkopf (12) mittels eines Tragarms (17) durch eine Öffnung (29) im weiteren Behälter (9) hindurch am Flüssigkeitsbehälter (6; 40; 44) angelegt ist.

24. Zusatzeinrichtung nach Anspruch 23, d a d u r c h g e k e n n z e i c h n e t , daß das durch die Öffnung (29) in das Innere des weiteren Behälters (9) ragende Ende des Tragearmes (17) eine U-förmige Trägerschiene (16) trägt, auf die der Ultraschallkopf (12) von außen durch die Öffnung (29) hindurch aufschiebbar ist.

25. Zusatzeinrichtung nach Anspruch 23 oder 24, d a - d u r c h g e k e n n z e i c h n e t , daß der Tragarm (17) aus mehreren Armteilen (18, 19, 20) besteht, die mittels Dreh- oder Verschiebegelenken (21, 22, 23) so relativ zueinander bewegbar sind, daß der Ultraschallkopf (12) im Innern des weiteren Behälters (9) in schräger Lage so am Flüssigkeitsbehälter (6; 40;

44) anlegbar ist, daß er im wesentlichen senkrecht zur Oberfläche des Körperteiles (4) Ultraschall-Sendesignale sendet und Echosignale empfängt.

26. Zusatzeinrichtung nach einem der Ansprüche 23 bis 25, d a d u r c h   g e k e n n z e i c h n e t , daß der Tragearm (17) an einer Drehwelle (25) für den weiteren Behälter (9) mittels eines Drehgelenks (23) befestigt ist.

27. Zusatzeinrichtung nach Anspruch 2, d a d u r c h g e k e n n z e i c h n e t , daß ein zylindrischer Körper (64) mit vertikal angeordneter Zylinderachse (68) vorgesehen ist, der eine schräg eingezogene, flüssigkeitsdichte Trennwand (70) enthält, welche den zylindrischen Körper (64) in einen oberen und einen unteren Behälter (62, 66) unterteilt, wobei der obere Behälter (62) als der Flüssigkeitsbehälter dient und zur Aufnahme der Ultraschall gut leitenden Flüssigkeit bestimmt ist, und wobei die Haltevorrichtung (84, 86) den Ultraschallkopf (50) innerhalb des unteren Behälters (66) an der Ankoppelstelle (80), die an der schräg eingezogenen Trennwand (70) liegt, festhält (Fig. 6 und 7).

28. Zusatzeinrichtung nach Anspruch 27, d a d u r c h g e k e n n z e i c h n e t , daß der Neigungswinkel (90° - $\alpha$ ) der Trennwand (70) bezüglich der Zylinderachse (68) etwa 70° beträgt.

29. Zusatzeinrichtung nach Anspruch 27 oder 28, d a - d u r c h   g e k e n n z e i c h n e t , daß als Trennwand (70) eine durch einen festen Boden (72) gestützte elastische Folie (74) vorgesehen ist, wobei im Boden (72) eine Ausnehmung (80) vorgesehen ist, innerhalb der die Abstrahlfläche (82) des Ultraschallkopfes (50) an der Folie (74) anliegt.

30. Zusatzeinrichtung nach einem der Ansprüche 27 bis 29, d a d u r c h   g e k e n n z e i c h n e t , daß die motorisch betriebene Einrichtung (82) zur Drehung der Haltevorrichtung und des Flüssigkeitsbehälters (62) unterhalb der Trennwand (70) angeordnet und mechanisch (94, 96, 98) mit dem Zylinder (64) zur Drehung desselben um die Zylinderachse (68) verbunden ist.

31. Zusatzeinrichtung nach Anspruch 1, d a d u r c h g e k e n n z e i c h n e t , daß ein Patientenauf- lagebrett (104) mit Durchtrittsöffnung (106) vorgesehen ist, durch die das zu untersuchende Körperteil (110) hindurchragt, daß die Durchtrittsöffnung (106) von einer flexiblen Membran (108) überspannt ist, an der das Körperteil (110) im Untersuchungsfall anliegt, daß als Flüssigkeitsbehälter eine Flüssigkeits-Vorlaufstrecke (112) vorgesehen ist, die als ein die Flüssigkeit enthaltender geschlossener Sack ausgebildet ist, wobei dieser Sack eine flexible Gleitfläche (116) und eine dieser Gleitfläche gegenüberliegende Auflagefläche (118) besitzt, die etwa die Größe der Abstrahlfläche (120) des Ultraschallwandlers (122) besitzt, daß die Auflagefläche (118) an die Abstrahlfläche (120) ultraschalldurchlässig und lösbar angekoppelt ist, daß die flexible Gleitfläche (116) an die Membranoberfläche (108) anlegbar ist, und daß der Sack (112) mit der Flüssigkeit mäßig gefüllt ist derart, daß sich die Gleitfläche (116) bei an der Membran (108) anliegendem Körperteil (110) der Form der Membran (108) und damit der Form des Körperteils (110) anpaßt.

32. Zusatzeinrichtung nach Anspruch 31, d a d u r c h g e k e n n z e i c h n e t , daß zum Einfassen des Sackes (112) eine Halterung (140) vorgesehen ist, die die flexible Gleitfläche (116a) freiläßt.

**0089682**

FIG 1

FIG 2

FIG 3

FIG 4

FIG 5

0089682
82 P 8510 Kb

FIG 6

FIG 7

0089682

FIG 8

FIG 9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-4 317 369 (S.A. JOHNSON / UNIVERSITY OF UTAH)<br><br>* Spalte 4, Zeile 41 - Spalte 6, Zeile 2; Abbildung 1 *<br><br>--- | 1-3,7-11,13,14,16-18 | A 61 B 10/00<br>G 10 K 11/00 |
| A | US-A-4 063 097 (D.M. BARRETT et al.)<br><br>* Spalte 5, Zeile 62 - Spalte 6, Zeile 59; Spalte 7, Zeilen 37-65; Spalte 8, Zeilen 34-42; Spalte 35, Zeilen 24-45; Spalte 39, Zeilen 25-40; Spalte 40, Zeilen 12-17; Abbildungen 1,2,18,19 *<br><br>--- | 1-3,7-11,13,14,18,20,21 | |
| A,D | EP-A-0 032 751 (W. IGL)<br><br>* Zusammenfassung; Seite 2, Zeilen 1-8; Seite 3, Zeile 31 - Seite 4, Zeile 3; Seite 4, Zeilen 31-35; Seite 8, Zeilen 12-22; Seite 9, Zeilen 6-18; Seite 9, Zeile 34 - Seite 10, Zeile 10; Seite 10, Zeile 26 - Seite 11, Zeile 7; Seite 12, Zeilen 17-32; Abbildungen 2,3,5,7 *<br><br>---              -/- | 1-7,12,13,18,19 | RECHERCHIERTE SACHGEBIETE (Int. Cl. ³)<br><br>A 61 B 10/00<br>G 10 K 11/00 |

Der vorliegende Recherchenbericht wurde für alle Patentansprüche erstellt.

| Recherchenort<br>DEN HAAG | Abschlußdatum der Recherche<br>29-06-1983 | Prüfer<br>RIEB D.K. |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | FR-A-2 425 837  (TOKYO SHIBAURA DENKI K.K.) <br> * Seite 2, Zeile 18 - Seite 3, Zeile 30; Abbildungen 1-3 * | 4,5,7, 18-21 | |
| A | US-A-4 130 112  (R.E. FRAZER) <br><br> * Zusammenfassung; Spalte 2, Zeile 52 - Spalte 3, Zeile 7; Spalte 3, Zeilen 23-31; Spalte 3, Zeile 53 - Spalte 4, Zeile 13; Spalte 4, Zeilen 24-38; Abbildungen 1-4 * | 15,18, 22 | |
| A | US-A-3 603 303  (J.R. STOUFFER) <br><br> * Zusammenfassung; Spalte 2, Zeilen 37-46; Spalte 3, Zeilen 3-11; Spalte 3, Zeilen 36-57; Spalte 4, Zeilen 54-75; Abbildungen 1-6 * | 1,16- 18,25, 31 | |
| A | DE-A-3 002 514  (LITTON) <br> * Seite 10, Zeilen 12-17; Abbildungen 1-4 * | 22 | |
| A | US-A-4 145 680  (R.W. SMITH) <br> * Zusammenfassung; Spalte 2, Zeile 52 - Spalte 3, Zeile 43; Abbildungen 1,2 * | 1 | |

RECHERCHIERTE SACHGEBIETE (Int. Cl. ³)

--- -/-

Der vorliegende Recherchenbericht wurde für alle Patentansprüche erstellt.

| Recherchenort <br> DEN HAAG | Abschlußdatum der Recherche <br> 29-06-1983 | Prüfer <br> RIEB D.K. |
|---|---|---|

**Europäisches Patentamt**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | US-A-3 496 764 (J.R. STOUFFER) <br><br> * Spalte 2, Zeilen 50-61; Spalte 4, Zeilen 11-23; Abbildungen 1,8 * <br><br> ----- | 1,31, 32 | |
| | | | **RECHERCHIERTE SACHGEBIETE (Int. Cl. 3)** |

Der vorliegende Recherchenbericht wurde für alle Patentansprüche erstellt.

| Recherchenort <br> DEN HAAG | Abschlußdatum der Recherche <br> 29-06-1983 | Prüfer <br> RIEB D.K. |
|---|---|---|